# EUROPEAN PATENT APPLICATION

(11) **EP 4 512 388 A1**
(43) Date of publication of application: **26.02.2025**
(21) Application number: 23792271.1
(22) Date of filing: 28.04.2023
(51) Int. Cl.: A61K 9/00, A61K 45/08, A61K 47/12, A61K 47/26, A61K 47/36, A61P 15/02

(54) **MEANS FOR INTRAVAGINAL DELIVERY OF AN ACTIVE INGREDIENT AND METHOD FOR PRODUCING AND USING SAME**

(30) Priority: 21.04.2022 RU 2022110775
(71) Applicant: OBSHCHESTVO S OGRANICHENNOJ OTVETSTVENNOSTYU «KINETIK-FARM», Solnechnogorsk, 141560 (RU)
(72) Inventor: LOZINSKIY, Vladimir Iosifovich, Moscow, 127576 (RU); IVANOV, Roman Viktorovich, Moscow, 115114 (RU); ZINCHENKO, Arkadiy Vladimirovich, Moscow, 127276 (RU); SHOBOLOV, Dmitriy L'vovich, Solnechnogorsk, 141560 (RU); STRYGIN, Alexey Sergeevich, Moscow, 129515 (RU)
(74) Representative: Osmans, Voldemars
(86) International application number: PCT/RU2023/050105
(87) International publication number: WO 2023/204734

(57) **Abstract**

The present invention relates to the chemical, pharmaceutical and medical industry and concerns the production of novel pharmaceutical dosage forms of medicinal products and medical devices suitable for vaginal delivery and used for preventing and treating diseases of the female reproductive system. The composition for producing an intravaginal delivery device for an active ingredient, and the said device, which is a biopolymer matrix without active ingredients comprises at least one structural polysaccharide selected from the group consisting of polysaccharides biodegradable in the vaginal cavity, at least one mucoadhesive component, and at least one pH modifier, which is an organic acid. The composition and the device may further comprise a selective microbial substrate. The composition for producing an intravaginal delivery device for an active ingredient, and said device may comprise at least one active ingredient in a therapeutically effective amount. The vaginal delivery device has a set of properties and characteristics that increase the treatment efficacy and ensure the device's ease-of-use.

## Description

### Technical field

The present invention relates to the chemical, pharmaceutical and medical industry and concerns the production of novel pharmaceutical dosage forms of medicinal products and medical devices suitable for vaginal delivery and used for preventing and treating diseases of the female reproductive system, including those associated with bacterial, fungal and viral infections caused by an imbalance in the microbiota, as well as menstrual cycle disorders, benign and malignant neoplasms of the genital organs, hyperplastic and dystrophic changes in the cervix, sexual dysfunction, reproductive dysfunction, menstrual dysfunction. The invention also relates to a method for vaginal delivery of various active ingredients. More specifically, the present invention is directed towards producing a cryostructured pharmaceutical dosage form for vaginal delivery of active ingredients.

### Prior art

Infectious diseases of the female reproductive system (including bacterial vaginosis, candidiasis, viral infections, as well as vaginal dysbioses) are one of the most common conditions that every woman faces at some point during her life. Both systemic antimicrobial agents and topical agents, including vaginal tablets, capsules, suppositories, ointments, gels and solutions, are used for treatment. In addition, topical antibiotic therapy is preferable compared to oral administration, since it reduces the risks of adverse events and side effects and increases the local concentration of antimicrobial agents. However, topical dosage forms known from the prior art such as suppositories, ointments, gels, solutions, etc., are characterized by disadvantages associated with the inconvenience of their use, irritation of the mucosa, itching, burning, etc., leading to non-compliance with the treatment regimen, which limits their use and often determines the choice in favor of systemic administration.

Features of the vaginal anatomy (an open-ended elongated cavity, resembling a tube approximately 10-12 cm long and 3-4 cm in diameter) [Nara Gevorkovna Zatikyan Anatomy of the vagina https://vmc.clinic/news/30/62/anatomiya-vlagalischa/d,article/], histological structure of the mucosa (ensuring continuous drainage of the vaginal cavity content, the so-called "self-cleaning" effect), biological properties of vaginal discharge (high viscosity and low volume) [Anderson B.L., Cu-Uvin S., Raker C.A. et al. Subtle perturbations of genital microflora alter mucosal immunity among low-risk pregnant women // Acta Obstet. Gynecol. Scand. 2011. Vol. 90, 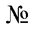 5. P. 510.], significantly limit the effectiveness of drugs used for vaginal administration based on traditional dosage forms (tablets, capsules, suppositories, ointments, gels and solutions).

Limitations of solid forms are associated with a suboptimal dissolution profile in the vagina and surface distribution of the therapeutic agent. Due to the small volume of fluid in the vagina (up to 1.5 mL), solid forms cannot provide the necessary concentrations of administered active substances or bacteria uniformly over the entire surface of the vaginal mucosa. There is an excess of the substance at the administration site of a tablet or capsule, which, however, is not further distributed over the entire mucosa, remaining only at the administration site. As a result, during treatment patients may experience vaginal dryness and a lack of internal fluid needed for tablet disintegration or capsule dissolution, or the dissolution of the capsule and the dissolution of the active substances contained in them. Sometimes a capsule or tablet with an active ingredient may fall out of the vagina without disintegrating, which adversely impacts the effectiveness of treatment. Information about capsules that have not dissolved can often be found in consumer reviews on product websites or online distributors of the respective drugs (for example, in reviews of FloraFemme intravaginal capsules [https://www.amazon.com/FloraFemme-Probiotic-Suppository-Antibiotics-Prevention/dp/B007HWMI6S], Lactogynal [https://03online.com/news/ne_rastvorilas_kapsula_laktozhinal/2018-1-1-373606 https://irecommend.ru/content/bespoleznaya-trata-deneg-131], Lactonorm [https: //03online.com/news/ne_rastvoryaetsya_laktonorm/2021-3-23-963866], Fluomizin vaginal tablets [https://health.mail.ru/consultation/2674492/ https://www.babyblog.ru/community/living/post/3182205]).

Semi-solid dosage forms, for example, suppositories, are more optimal in terms of uniform distribution of therapeutic agents throughout the vaginal mucosa, including probiotics and antimicrobial agents; however, the suppository mass or ointment base that has melted at body temperature often leak from the vagina. The use of gels and solutions containing antimicrobial agents ensures even more uniform distribution throughout the vaginal mucosa; however, these forms are also prone to leakage, requiring a horizontal position before and after administration, which reduces compliance with therapy, and, as a consequence, the effectiveness of therapy. In addition, fatty bases of semi-solid dosage forms have limitations in terms of introduction of a wide range of hydrophilic substances or bacteria, and aqueous bases have limitations in terms of the stability of the active substances introduced into their composition and require the simultaneous introduction of preservatives, which adversely affects the physiology of the vagina.

Thus, the use of conventional solid and semi-solid dosage forms for intravaginal administration of drug substances and probiotic microorganisms is associated with a number of problems that adversely affect the effectiveness of therapy, ease of use and patient adherence to long courses of therapy. Many patients do not complete the initiated treatment.

Relevant is the development of a specialized dosage form for intravaginal administration of the active ingredient, taking into account the physiological and morphological characteristics of the vagina and simultaneously having the advantages of solid dosage forms (stability during storage, versatility with respect to various therapeutic agents, ease and convenience of administration, dosing accuracy) and the advantages of semi-solid dosage forms (optimal and uniform distribution of the therapeutic agent throughout the vaginal mucosa, comfortable sensations). Such dosage form should provide create physiologically favorable conditions in the vaginal environment after administration (for example, pH, osmolality) to reduce adverse reactions at the injection site, such as burning, itching, irritation and swelling. It is also preferable to maintain a healthy vaginal microbiome during and after antimicrobial therapy, for example, through the use of selective prebiotics that provide a nutrient-rich environment for vaginal lactobacilli and are not a growth substrate for opportunistic microflora.

One of the directions for creating such dosage forms is the development of cryostructured lyophilized vaginal dosage forms in the form of tablets and inserts. The closest solutions to the claimed group of inventions are the following intravaginal dosage forms.

The vaginal dosage form presented in the article (B. Vitali et al. Vitali et al. Association of Lactobacillus crispatus with fructo-oligosaccharides and ascorbic acid in hydroxypropyl methylcellulose vaginal insert/Carbohydrate Polymers 136 (2016) 1161-1169), which describes a probiotic agent in the form of a vaginal insert (insert), which is a hydroxypropyl methylcellulose (HPMC) matrix as a structural and mucoadhesive polymer containing skimmed milk powder, fructo-oligosaccharides, ascorbic acid and *L. Crispatus* lactobacilli. The presented probiotic device is produced by dissolving the ingredients in water, freezing and freeze-drying. In dry form, the device is a porous cylinder with a diameter of 8.0-8.7 mm and 2224 mm long. When placed in an aqueous solution, the vaginal insert absorbs water, increasing in volume fivefold. The swollen device is capable of gradually releasing encapsulated lactobacilli. The disadvantage of this vaginal insert is the slow absorption of water by the matrix (up to 3-4 hours) due to the use of HPMC as a structural polymer, which can lead to a slower onset of the therapeutic effect compared to the claimed device, particularly in conditions of a small volume of fluid in the vagina and does not fundamentally address the issue of slow disintegration of the insert upon vaginal insertion.

An article by Abruzzo, A., Bigucci, F., Cerchiara, T., Saladini, B., Gallucci, M. C., Cruciani, F., et al. (2013). Chitosan/alginate complexes for vaginal delivery of chlorhexidine gluconate. Carbohydrate Polymers, 91(2), 651-658 describes a lyophilized vaginal insert based on a polyelectrolyte complex of chitosan and alginate as a structural matrix, mannitol to enhance the lyophilizate structure and chlorhexidine digluconate. The disadvantage of this solution is the use of a polyelectrolyte complex as a structural component, which, from a functional standpoint, noticeably delays the release of the therapeutic agent (not exceeding 20-30% within 6 hours) compared to neutral matrices due to electrostatic interaction with the matrix of therapeutic agent ions. In addition, this solution is characterized by a more complex process for producing the insert compared to the solution described above and the claimed solution.

The closest solutions to the claimed group of inventions are a pharmaceutical composition and a vaginal delivery device based on it (WO 2018/069888) in the form of a lyophilized tablet, which contains gelatin as the main mucoadhesive component (alone or in combination with other mucoadhesive polymers), mannitol as a structural component, a pH modifier for creating an optimal value for the vaginal environment, and acyclovir as a therapeutic agent. The described form is characterized by a porous structure, rapid swelling and disintegration in water and aqueous solutions in less than two minutes. The tablet is prepared by dissolving gelatin and mannitol in an aqueous solution, dissolving or dispersing the therapeutic component in the resulting solution, filling into forms and subsequent lyophilization. Less than 1 mL of liquid is required for hydration and disintegration of the tablet, which is the optimal amount for vaginal delivery and solves the issue of slow disintegration of vaginal tablets. This form has a number of advantages over the known forms (tablets) intended for local vaginal delivery of a therapeutic agent; it provides a more predictable absorbed dose of the therapeutic agent during intravaginal administration, but is less effective for local delivery and uniform distribution of the therapeutic agent over the entire surface of the vaginal mucosa compared to semi-solid dosage forms (gels, ointments, suppositories) due to a relatively small size. In addition, administration of a dry tablet is less convenient and physiological compared to other forms, and the possibility of wetting the tablet to facilitate administration is limited by its rapid disintegration upon contact with aqueous solutions. An important disadvantage of gelatin-containing intravaginal dosage forms is that this protein substance, i.e. gelatin may serve as an accessible substrate for pathogenic microflora if present, while intravaginal lactobacilli do not consume gelatin. Thus, administration of such tablets into the vagina will promote the growth of undesirable microorganisms.

The technical problem solved by the claimed invention is the development of a new form of matrix (a device without a therapeutic agent) and a dosage form (a device containing a matrix with a therapeutic agent) intended for vaginal administration in the prevention and treatment of diseases of the female reproductive system associated with bacterial, fungal and viral infections caused by an imbalance in the microbiota, and ensuring effective delivery, rapid disintegration in the vagina, maximum and uniform distribution of the therapeutic agent in the vaginal cavity, insignificant leakage from the vaginal cavity, sufficient exposure time increasing the effectiveness of therapy, while simultaneously ensuring physiological and convenient use of the device, which increases adherence to therapy.

### Summary of invention

The technical result of the group of inventions is the production of a solid dosage form (device) for vaginal administration, based on the use of a biodegradable polysaccharide as a structural component, which has the following set of properties and characteristics that increase the effectiveness of therapy while ensuring ease of use of the device:
1) In the initial state (before contact with water or an aqueous solution before inserting the device into the vagina), the device has a stable solid form, for example, resembling a hygienic tampon, convenient for insertion into the vagina;
2) Upon interaction with water or an aqueous solution, the device is effectively wetted, forming a three-dimensional hydrogel structure while retaining its original shape for up to 5 minutes, ensuring hygienic insertion of the device into the vaginal cavity manually or using a suitable applicator device;
3) Upon subsequent intravaginal mechanical action (compression due to the physiological elasticity of the vaginal walls), the hydrogel disintegrates forming a gel-like mass that ensures the uniform distribution of the therapeutic agent in the vaginal cavity;
4) The device is characterized by the necessary adhesion to the walls of the vaginal epithelium, providing sufficient exposure time to the therapeutic agent in the vaginal cavity;
5) The device is characterized by structural strength in the initial state (before contact with water or an aqueous solution), and provides nutritional support for lactobacilli of the vaginal microflora after insertion of the device;
6) When the device is wetted in water, the pH level within pH 4.5 ± 0.6 is ensured.

The parameters of the claimed intravaginal delivery device for an active ingredient are presented in more detail in Table 1.

**Table 1.**

| No. | Parameter | Parameter value |
|---|---|---|
| 1. | Weight and size characteristics of the device in its original state (length, mm/diameter, mm/weight, g) | 20-100/5-20/0.1-2.5, preferably 30-60/5-15/1-2 |
| 2. | Average pore size, µm | 5-100, preferably 10-70 |
| 3. | Specific pore density, g/cm³ | 0.1-0.3 |
| 4. | Swelling capacity (weight of swollen device/weight of dry device) | 4-8, preferably 4.5-6.5 |
| 5. | Hydration time (swelling time to maximum weight), min | 0.5-5 min |
| 6. | Release time of the active ingredient (time to release >80%), min | 5-60 min |
| 7. | Time during which the device retains its initial shape after interaction with water or an aqueous solution, min | up to 5 min |
| 8. | Mechanical strength in a dry state (breaking stress limit during compression crushing), N | from 2 to 10, preferably from 2 to 6 |
| 9. | Moisture content, %: | |
| | Device without probiotic microorganisms | up to 5 |
| | Device with probiotic microorganisms | up to 9 |
| 10. | Survival of lactobacilli cells in the device, % | 30-70 |

The technical result is achieved by using an intravaginal delivery device (or medical device) for an active ingredient, which is a new pharmaceutical dosage form based on a biopolymer matrix. The device in one of the embodiments may not contain active ingredients, i.e. may be an unfilled biopolymer matrix, which can also be used as a finished therapeutically active medical device. In another embodiment of the invention, the device is a biopolymer matrix containing one or more active ingredients, wherein the active ingredients can be introduced into the matrix at the initial stage of producing the device: when mixing the matrix components with the active ingredients before freezing and freeze-drying, and at subsequent stages of production or use of the finished product.

The composition for producing of an intravaginal delivery device for an active ingredient, and the said device, which is a biopolymer matrix without active ingredients comprises at least one structural polysaccharide selected from the group consisting of polysaccharides biodegradable in the vaginal cavity, at least one mucoadhesive component, and at least one pH modifier, which is an organic acid. The composition and the device may further comprise a selective microbial substrate.

The composition for producing of an intravaginal delivery device for an active ingredient, and said device may comprise at least one active ingredient in a therapeutically effective amount. The active ingredient may be selected from the group consisting of antibacterial agents, including macrophages, and/or antiseptic agents, and/or antifungal agents, and/or antiretroviral agents, as well as hormones, cytostatic agents, vaccines intended for intravaginal administration. In addition, probiotic microorganisms, including lactobacilli, consortia of probiotic microorganisms for intravaginal administration can be used as an active ingredient, wherein selective substrates for microorganisms can be additionally included in the composition and the device.

Possible options of the formulation of the initial composition for producing the claimed device and the composition of the resulting device are presented in Table 2.

**Table 2.**

| Ingredient | | | Per 1 mL of water or aqueous solution of the composition (before lyophilization) | Weight per 1 g of device (after lyophilization) |
|---|---|---|---|---|
| Structural polysaccharide (one or more), mg | | | from 40 to 100 preferably from 60 to 100 | from 400 to 700 preferably from 500 to 700 |
| Mucoadhesive component (one or more), mg | | | from 10 to 40 preferably from 10 to 30 | from 150 to 300 preferably from 150 to 250 |
| pH modifier (required pH: pH 4.5 ± 0.6) - organic acid (one or more), mg | | | from 10 to 30 preferably from 10 to 25 | from 50 to 100 preferably from 50 to 75 |
| Selective substrate (one or more), mg | | | from 0 to 40 preferably from 20 to 40 | from 0 to 250 preferably from 100 to 250 |
| Active ingredient, mg including: | | | | |
| | antibacterial agents | | from 0 to 50 | from 0 to 250 |
| | | | preferably 5-50 | preferably 30-250 |
| | antiseptic agents | | from 0 to 10 | from 0 to 70 |
| | | | preferably 0.1-10 | preferably 0.5-70 |
| | antifungal agents | | from 0 to 50 | from 0 to 250 |
| | | | preferably 5-50 | preferably 30-250 |
| | antiretroviral agents | | from 0 to 50 | from 0 to 250 |
| | | | preferably 10-50 | preferably 50-250 |
| | hormones | | from 0 to 2 | from 0 to 15 |
| | | | preferably 0.02-2 | preferably 0.2-15 |
| | cytostatic agents | | from 0 to 50 | from 0 to 250 |
| | vaccines, incl. | | preferably 5-50 | preferably 20-200 |
| | | • Bacterial lysates | from 0 to 1 mg | from 0 to 10 |
| | | | preferably 0.01-0.8 | preferably 0.1-8 |
| | | • Antigens (protein, lipopolysaccharide) | from 0 to 0.1 mg | from 0 to 1 mg |
| | | | preferably 0.01-0.05 | preferably 0.1-0.5 |
| | | • Live attenuated viral particles | from 10⁷ to 10¹¹ particles | from 10⁸ to 10¹² particles |
| | | | preferably from 10⁸ to 10¹⁰ particles | preferably from 10⁹ from to 10¹¹ particles |
| Active ingredient: | | | from 0 to 1×10⁹ | from 0 to 1×10⁹ |
| | probiotic microorganisms, CFU | | preferably 1×10⁶ - 1×10⁹ | preferably 1×10⁷ - 1×10⁹ |
| Water | | | 1 mL | removed by lyophilization to a residual content of not more than 9% |

The structural polysaccharide is selected from the group consisting of polysaccharides that are biodegradable in the vaginal cavity, preferably polysaccharides in which glucose molecules are linked to each other predominantly through α-(1→4) and α-(1→6) glycosidic bonds, for example, starch, amylopectin, dextrins, polydextrose.

The mucoadhesive component may be selected from the group consisting of hydroxypropyl cellulose, carboxymethylcellulose, hyaluronic acid, guar or xanthan gum.

As the organic acid, an acid suitable for intravaginal administration may be used, including ascorbic, pyruvic, malic, lactic or succinic acid etc.

The selective substrate is preferably selected from the group consisting of oligosaccharides biodegradable in the vaginal cavity, preferably for intravaginal lactobacilli, the chain of which contains about 2-20 monosaccharide units, for example, lactulose, raffinose, maltodextrin, oligofructose, isomaltooligosaccharide, inulin, glycogen.

The intravaginal delivery device for an active ingredient is designed as a voluminous, oblong, solid, dry body, which, in one embodiment, may resemble the shape of a feminine hygiene tampon. The device is produced from biodegradable polysaccharides and is characterized by the parameters presented in Table 1, including the ability to absorb liquid (aqueous solutions) in an amount required to fill the internal volume of interconnected (communicating) macropores with the formation of a hydrogel that retains its initial shape for a certain time (up to 5 minutes), and characterized by a strength sufficient for insertion of the device into the vaginal cavity.

The claimed group of inventions also comprises:
A method of producing of a therapeutic agent for vaginal administration, comprising the preparation of aqueous solutions of the initial composition ingredients, mixing thereof, freezing the resulting mixture at a temperature selected from the range from minus 15 °C to minus 30°C for 3-18 hours with subsequent freeze-drying.

The method for intravaginal delivery of an active ingredient for the treatment and/or prevention of diseases of the female reproductive system comprises insertion of the resulting device into the vagina, wherein the device, before being inserted into the vagina, is preliminarily held (up to 5 minutes) soaked with water or an aqueous solution until a hydrogel is formed. Female reproductive system diseases include bacterial vaginosis; purulent inflammatory diseases (vulvitis, colpitis, endometritis, adnexitis, pelvic peritonitis), sexually transmitted infections (gonorrhea, trichomoniasis, chlamydiosis, ureaplasmosis, candidiasis, genital herpes, papillomavirus infections, cytomegalovirus infection, HIV infection), benign and malignant neoplasms of the genital organs, fibroids, hyperplastic and dystrophic changes in the cervix (erosions and pseudo-erosions), sexual dysfunction (pain during intercourse), reproductive dysfunction (miscarriages, premature birth, infertility), menstrual dysfunction (oligomenorrhea or hypermenorrhea, amenorrhea, menorrhagia, dysmenorrhea, anovulation), etc.

The composition and concentration of the ingredients of the polymer composition, on the basis of which the claimed device (medical device) is produced, was found experimentally and is determined by the following factors.

A class of polysaccharides in which glucose molecules are linked to each other through α-(1→4) and α-(1→6) glycosidic bonds was selected due to their biocompatibility and lack of irritation to the mucous membranes, on the one hand, and biodegradability by vaginal lactobacilli. The use of native starch (for example, corn, potato, tapioca starches, sago, etc.) or biodegradable modified starch is envisaged as a structural polysaccharide. For example, glycogen, the closest structural analogue of plant starch, is the primary nutrient substrate for vaginal lactobacilli flora. Unlike pathogenic microorganisms of the vagina, lactobacilli are capable of fermenting starch, and therefore it is a selective prebiotic for lactobacilli. The use of other classes of biocompatible polysaccharides as a structural component (for example, some cellulose-based polymers) does not lead to effective release of encapsulated therapeutic agents due to the non-biodegradability of the matrix.

The claimed concentration range of the structural polysaccharide in such a composition is within the range of 40 to 100 mg/mL. At values of this component below 40 mg/mL, undesirable cracking of samples often occurs during freeze-drying, and at concentrations above 100 mg/mL, the viscosity of the initial polymer composition greatly increases, which creates technical difficulties in manipulating it, especially in terms of uniform distribution of insoluble components (for example, lactobacilli, antibacterial and antifungal agents) included in the polymer matrix. The specified limits are also optimal with respect to the properties of the resulting matrix. At a low concentration of the structural polysaccharide, a hydrogel with low strength is formed; at a high content of this component, the strength of the hydrogel is too high for effective degradation inside the vagina.

To ensure prolonged retention of hydrogel particles in the vaginal cavity, the dosage form composition includes at least one mucoadhesive polymer. The introduction of a mucoadhesive component into the composition of the device improves adhesion to the vaginal epithelium, which was demonstrated during model experiments on acrylamide gel. Substances of a polysaccharide nature, including hydroxypropyl cellulose, carboxymethylcellulose, hyaluronic acid, gelatin, agarose, sodium alginate, guar and xanthan gums, were used as mucoadhesive components in the experiments. The best adhesive properties in the composition with a structural polysaccharide and a selective substrate were exhibited by hydroxypropyl cellulose, carboxymethylcellulose, hyaluronic acid and guar gum, while the first two polymers are not bioresorbable polysaccharides and will be gradually eliminated from the female body with natural secretions, hyaluronic acid and guar gum are biodegradable by enzymes located in the vaginal canal.

The optimal amount of this component for various additives is 10-40 mg per 1 mL of water or aqueous solution in the initial composition or 150-300 mg per 1 g of the end product, a dry cryostructured form, was selected experimentally. Using a smaller amount of this component resulted in weak mucoadhesive properties of the proposed device. Using a larger amount of the mucoadhesive component (exceeding the specified upper limit of the presented range) made it difficult to introduce it into the composition due to process limitations by viscosity of the polymer solution prepared before cryostructuring.

To achieve the physiological acidity of the intravaginal environment, organic acid is used in the matrix, in particular, ascorbic, pyruvic, malic, succinic, lactic, etc., in the amount necessary to ensure the pH after wetting the dry dosage form with water within pH 4.5 ± 0.6. These particular acids were selected due to their solid phase state in the claimed device, non-volatility during lyophilization, low hygroscopicity and rapid solubility in an aqueous environment when using the device. The concentration range of these acids was determined experimentally by the acidity of the composition before lyophilization, which must fit within the above values.

The presence of a selective substrate in the composition of the oligosaccharide-based device, in addition to the structure-forming agent biodegradable by lactobacilli, ensures rapid growth of lactobacilli after the introduction of a probiotic culture, or prebiotic support of the lactobacilli pool during antibiotic therapy. It is known that the molecular weight of φ polysaccharide affects the rate of fermentation by lactobacilli. Moreover, shorter poly- and oligosaccharides are preferable to longer polymer chains. On the other hand, the introduction of a significant quantity of disaccharides into the biopolymer matrix turned out to be suboptimal in terms of forming a gel structure with good mechanical properties. Thus, as a result of the conducted studies, it was determined experimentally that the optimal chain length of oligosaccharides is about 2-20 monosaccharide units per chain, resulting in a stronger structure of the dry form, ensuring convenience of its production. The introduction of oligosaccharides of this chain length ensures rapid growth of lactobacilli, compared to a matrix without oligosaccharides. With that, unlike introduced disaccharides (for example, maltose and lactulose), the device retains its solid gel structure with specified mechanical properties. In the claimed technical solution, an oligosaccharide selected from the group of lactulose, raffinose, maltodextrin, oligofructose, isomaltooligosaccharide, and inulin, is used as a selective substrate for intravaginal lactobacilli. It is also possible to additionally introduce glycogen. The optimal amount of substrate in the initial composition is 10-40 mg per 1 mL of water or aqueous solution, in the finished dry cryostructured form - from 100 to 250 mg per 1 g of device, which ensures optimal growth of lactobacilli culture (for example, *Lactobacillus Crispatus*) without compromising the mechanical properties of the device. The experiment showed that at a substrate content less than 20 mg/mL, the proliferation rate of the corresponding lactobacilli decreased, and at a substrate concentration above 40 mg/mL, a decrease in the hydrogel strength was observed after wetting of the lyophilized sample, which may be due to both the interaction between the chains of the structural polymer and substrate, and the high osmotic pressure created by the dissolved substrate within the hydrogel.

In the claimed technical solution, various lactobacilli, preferably obtained from vaginal strains, can be used as the therapeutic agent at a dose of 1×10⁶ - 1×10⁹ CFU/cm³ of device. In various probiotic preparations for intravaginal administration, a single dose of probiotics used is usually from 10⁸ to 10⁹ CFU. Thus, strengths within these limits can be considered optimal to achieve a clinically significant effect. When choosing the limits for the content of medicinal substances in the initial composition and the device, typical strengths used in drug products for intravaginal administration were considered. With that, depending on the size and volume of the device, the dose of the administered drug substance will not exceed the dose of these substances used in standard gynecological practice.

It is advisable to produce the device as an oblong body, which, for example, may resemble a feminine hygienic tampon. This form has proven itself well in feminine hygiene devices, due to ease of insertion and positioning inside the organ, good retention, and comfort for the woman. With that, the invention allows producing devices of any shape and size without significant changes in technology.

To ensure long-term stability of the therapeutic agent in the polymer matrix, the presence of water throughout the storage period is undesirable. Lyophilization is used to remove water, which results in the formation of a solid, rigid, porous structure. This structure of the biopolymer matrix is formed by a structural biopolymer, which, upon subsequent wetting of the freeze-dried matrix in water, does not completely dissolve, but forms a hydrogel that has an internal structure and a stable external shape. Thus, the biopolymer solution undergoes cryostructuring when the solution is frozen followed by freeze-drying. Solutions of glucose-based structural biopolymers linked by alpha-glycosidic bonds (for example, starch) form gels (cryogels) as a result of cryogenic treatment and, thus, can be used as structure-forming agents. At the same time, for example, hydroxypropyl methylcellulose solutions do not form gels as a result of cryogenic treatment. After freeze-drying and re-moistening of the matrix, it completely dissolves in water, which does not allow the use of a device containing such a structure-forming agent for insertion into the vagina without an injector. Insertion of such a device in dry form is extremely difficult, uncomfortable, and can also be traumatic for the vaginal walls.

Due to the high porosity and sponge-like structure of the biopolymer matrix, when the device is moistened with water, it quickly absorbs it, due to which it becomes a reservoir of liquid in which the introduced therapeutic agents are dissolved or dispersed, ensuring their release. This property favorably distinguishes the use of the claimed device from other solid dosage forms such as tablets or capsules, which, after insertion into the vagina, dissolve slowly in a limited amount of vaginal fluid, without ensuring uniform distribution of the active ingredient over the entire internal surface of the organ. Thus, due to the use of the qualitative and quantitative composition of the biopolymer matrix components, as a result of preliminary wetting of the claimed device before its insertion into the vagina, a hydrogel forms that retains the shape of the initial solid device and has a slick surface, which ensures its comfortable insertion into the vaginal cavity. With that, the mechanical properties of the resulting hydrogel (breaking stress limit during compression crushing, see Table 1) are sufficient for convenient hygienic insertion into the vagina, for example, using a vaginal applicator. After insertion into the vaginal cavity, the hydrogel deforms due to shear stresses acting on it from the vaginal walls, which have physiological elasticity, gradually turning into the consistency of a regular (soft) gel, evenly covering the surface of the mucosa, without leaking from the vaginal canal. Lower values of breaking stress lead to the destruction of the hydrogel formed upon wetting of the device in the hands or applicator, which makes insertion into the vagina difficult or impossible. Higher levels do not provide rapid deformation of the hydrogel and transition to the consistency of a soft gel in the vagina, which slows down the release of the therapeutic agent and does not ensure effective distribution of the device throughout the mucosa.

The listed properties of the intravaginal delivery device (medical device) for an active ingredient-a biopolymer matrix without an active ingredient and a biopolymer matrix with an active ingredient-are implemented through the use of the qualitative and quantitative composition of the biopolymer matrix components, as well as the parameters of the method of producing the device, characterizing the freezing regimen of the initial composition for subsequent freeze drying.

The resulting properties of the device (listed in Table 1) provide more convenient and effective use compared to the dosage form adopted as a prototype. Unlike the prototype, the device is administered as a swollen hydrogel containing a large volume of liquid, which ensures, after the hydrogel disintegration in the vaginal cavity, a more uniform distribution compared to the prototype, and comparable to ointments and suppositories, and unlike the latter, the hydrogel does not have fluidity and can remain in the vaginal cavity for a long time.

The claimed device (biopolymer matrix and medical device or device based on it) has a number of advantages compared to known medical devices and means used for insertion into the vagina for the prevention and/or treatment of the female reproductive system diseases.
1) Compared to solid dosage forms (tablets, capsules):
   a. Dissolution of a therapeutic agent (hydrophilic) or preliminary rapid revival of freeze-dried microorganisms (when introducing, for example, probiotics) by wetting the device in water immediately before administration, which increases its bioavailability for the vaginal mucosa;
   b. Faster and more uniform distribution of the hydrogel throughout the mucous membrane, which increases the area of action of and the exposure time to the administered therapeutic agent on the mucosa;
   c. Comfort during insertion and placement of the device in the vaginal canal.
2) Compared with semi-solid and liquid dosage forms (gels, suppositories, ointments, solutions):
   a. It can be used for a wide range of therapeutic agents, including live microorganisms;
   b. It does not have fluidity, does not leak from the vagina;
   c. It ensures the accuracy of dosing of administered therapeutic agents;
   d. It ensures stability during long-term storage without the use of preservatives.

Thus, the use of the proposed device combines the advantages of solid and liquid vaginal dosage forms, eliminating their inherent disadvantages.

Other therapeutic agents in addition to those listed above, for example, vitamins, tissue regeneration stimulators, medicinal plant extracts, or preparations based on them, etc., can be added to the initial composition and device.

In some cases, in each of the above-described embodiments of the invention, the claimed device may additionally comprise one or more additional substances included in the matrix, which may be lubricants, fillers, preservatives, binders, stabilizers, emulsifiers, solubilizers, dyes, fillers and osmotic pressure regulators.

### Brief description of drawings

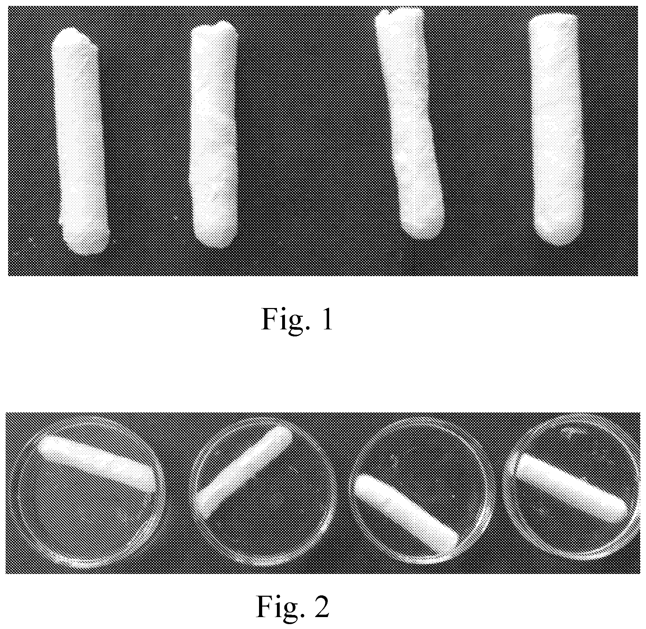

The invention is illustrated by photographs, where Fig. 1. presents photographs of dry samples obtained in examples from left to right: 2, 3, 6 and 9; Figure 2 shows photographs of swollen samples after 100 seconds of holding in water, obtained in examples from left to right: 2, 3, 6 and 9.

### Description of embodiments

The following is a detailed description of the claimed invention. The description of the embodiments is explanatory, demonstrating the possibility of achieving the claimed technical result. This technical solution may be subject to various changes and modifications that can be understood by a person skilled in the art upon reading this description. Such changes do not limit the scope of the claims. For example, the composition of the ingredients, their ratio, their combinations, weight and size characteristics, the choice of the active ingredient, as well as technological regimens for producing the device, which depend on the composition of the selected ingredients, may change. In addition, the claimed device can be used to immobilize a wide range of active components.

The intravaginal delivery device for an active ingredient according to the present invention is a solid lyophilized vaginal dosage form comprising ingredients in the ranges listed in Table 2.

The method for obtaining the claimed device (medical device) comprises the following operations (stages):
1) An aqueous solution is prepared from a mixture of a matrix-forming polysaccharide, a selective substrate of microorganisms, and a mucoadhesive component.
2) One of the above organic acids is introduced into the resulting solution to obtain a solution with the required pH values.
3) If necessary, an active component (therapeutic agent) or several such components are introduced into the solution. When probiotic microorganisms in the device composition, dry cells of these microorganisms, for example, lactobacilli, are dispersed in the resulting solution, or a solution or suspension of the medicinal substance is introduced.
4) The prepared polymer composition is transferred into injection molds of the required volume and geometry, then frozen at a temperature selected from the range of minus 15 °C to minus 30 °C for 3-18 hours and then freeze-dried.

The temperature and time regimes of cryogenic processing of the polymer composition obtained at stage 3 were determined experimentally. The upper limit of this range is due to the fact that at temperatures higher than minus 15 °C, the polymer compositions of the claimed formulation often do not freeze due to the effects of overcooling, and the use of temperatures below minus 30°C is impractical for economic reasons due to increased energy costs.

The cryostructured biopolymer matrix of the proposed medical device is a three-dimensional macroporous material produced from an aqueous solution of selected polymers of the composition of the specified formulation as a result of its freezing and subsequent lyophilization. A dry macroporous polymer material formed according to this scheme retains its shape well, does not exhibit fluidity, and has high stability, which is usually characteristic of dry materials. As initial polymers for its formation, the present invention involves the use of various polysaccharides, including starch and its derivatives, glycogen, while dextrins, maltodextrins, oligofructose, inulin, as well as other related biopolymers are used as a selective substrate for intravaginal lactobacilli.

Freeze-drying of a frozen initial polymer composition results in a solid, highly porous material of spongy morphology with an average pore cross-section of 5-100 µm and a specific density of 0.1-0.3 g/cm³, capable of very quickly (within a few seconds to several minutes) absorbing water in the amount required to fill the internal volume of interconnected (communicating) macropores to form a hydrogel with the initial shape and specified mechanical properties. In dry form, polysaccharide matrices ensure the stability of therapeutic agents in the composition of the claimed medical device, have good protective properties against microorganisms and ensure long-term preservation of their viability. The inclusion of hydroxypropyl cellulose, carboxymethylcellulose, hyaluronic acid, guar gum or xanthan gum in the matrix makes the target medical device highly mucoadhesive to the epithelium, which ensures long-term presence in the vaginal cavity. With that, biopolymers of the cryogenically structured matrix are selected in such a way that its polysaccharide base acts as a substrate for microflora bacteria and completely degrades in the female body.

Before use, the claimed therapeutic agent is usually wetted in water or an aqueous solution to saturate it with liquid, after which the device can be inserted into the vaginal cavity using fingers or an applicator. When placed in aqueous solutions before insertion into the vagina, the device absorbs liquid and swells, and in the swollen state it is a hydrogel (hydrated polymer system) whose slick surface, unlike the dry form, ensures comfortable insertion and stay in the vaginal cavity. In the vagina, on the one hand, mechanical deformation, destruction and grinding of the hydrogel occurs, and on the other, the structural polysaccharide of the matrix is biodegraded by vaginal microorganisms. The method of administration can be selected by the user depending on their own preferences. In some cases (for example, abundant discharge), it is possible to administer a therapeutic agent without prior wetting or moistening, but in this case, it is advisable to use an applicator to minimize trauma to the vaginal mucosa during administration. With that, the rapid saturation of the device with liquid occurs due to the excessive secretion of vaginal discharge.

Thus, the resulting device can act as a finished medical device, which can be inserted into the vagina using a non-applicator method or an applicator method (through a special tube). In the latter case, the medical device can be a device placed in a corresponding applicator, which in one embodiment can be made of two elements: an external perforated holding tube (cylinder) and an internal ejector rod (piston). The tube is perforated to facilitate the entry of water into the device when the applicator with the device in it is immersed in water or aqueous solutions. The device before its insertion in such a medical device can be moistened in any convenient way, including placing it in a vessel with water or saline solution, filling an applicator with water or solution with the device inserted inside, or another suitable and acceptable method for the user. Applicators can be made of any acceptable material (polymer or cardboard) and have design similar to a syringe. The outer tube usually has a smooth surface to facilitate administration of the device and may have a rounded end with petals to hold the device in the applicator before its insertion, or may be perforated. The nature of the perforation in terms of size, shape, location and number of holes is selected in such a way as to ensure free access of liquid to the device during wetting.

The implementability of the invention with the implementation of the claimed purpose is confirmed, but not exhausted by examples 1-26 presented in Table 3.

The parameters of the method of producing the device according to examples and the characteristics of the resulting device are presented in Table 4.

The devices according to examples 1-24 were produced by preparing aqueous solutions of the components used: a structural polysaccharide, a selective substrate, a mucoadhesive component, followed by mixing them and introducing a pH modifier into the resulting mixture. The devices according to examples 1-21 were produced using active ingredients, and the active ingredients were introduced into the mixture before the start of cryostructuring; in examples 25, 26, the active components were introduced after cryostructuring. With that, the resulting device was immersed in an aqueous solution of the active ingredient and kept at room temperature for 3 minutes until the device was saturated with the specified therapeutic agent.

In particular, when using probiotic microorganisms as an active ingredient (examples 1-9 and 16-18), dry microorganism cells were dispersed in a mixture of aqueous solutions of a structural polysaccharide, a selective substrate, and a mucoadhesive component. The prepared suspension was dosed into injection molds, frozen and freeze-dried. The resulting dry form of the device was a cylindrical rod with a rounded end (see Fig. 1), having a porous spongy texture, containing lactobacilli cells enclosed in a polysaccharide matrix.

The properties of the intravaginal delivery devices for active ingredients produced according to examples 1 to 26 were studied; the results of the studies are presented in Table 4.

### Swelling capacity and hydration time

The hydration time and swelling properties of the device were studied by placing samples of the device weighing 0.5 g in an excess of water of 10 mL. Then, every 30 seconds, one of the samples was removed and weighed. Swelling was determined as the ratio of the weight of the wetted sample to the dry sample after reaching the limit of the weight for the wetted sample. The swelling properties of all samples from examples 1-21 varied in the range of 4-8. The maximum mass of the hydrated sample was reached in the range of 30 seconds to 5 minutes after placement in an aqueous environment.

### Release of drug substances from the device

To study the release of drugs, a dry sample weighing 0.5 g was placed in a vial with 200 mL of NaCl solution (0.9 wt%) and left to incubate on a shaker (speed 40 rpm) at 37 °C. At certain intervals, samples were taken from the vial and the concentration of the drug substances in the solution was measured by HPLC. In all experiments, a fairly rapid release of drug substances from the polymer matrix of the device was observed: for devices from examples 10, 11, 14, 15, more than 80% of the included chlorhexidine digluconate, miramistin, clindamycin and tenofovir released within 5 minutes; for devices from examples 12 and 13, more than 80% of the included metronidazole and fluconazole released within 60 minutes.

### Release of lactobacilli from the device

To study the release of lactobacilli, a dry sample weighing 0.5 g was placed in a Petri dish with 20 mL of buffer simulating vaginal fluid pH 4.2 (composition per 1 L: 3.51 g NaCl, 1.40 g KOH, 0.222 g Ca(OH)₂, 0.018 g bovine serum albumin, 2 g lactic acid, 1 g CH₃COOH, 0.16 g glycerol, 0.4 g urea and 5 g glucose) and left to incubate on a shaker at 37 °C. At certain intervals, samples were taken from the Petri dish and the concentration of microorganisms in the medium was measured turbodimetrically by the absorption of the solution at 600 nm. For products from examples 1-8, the increase in solution turbidity occurred almost linearly until reaching a plateau corresponding to the complete release of microorganisms from the biopolymer matrix, and the time to plateau ranged from 4 to 8 hours.

### Survival of encapsulated bacteria in the device

The count of viable bacteria was determined immediately after encapsulation and production of the device and during storage at +2-8 °C and 25 °C for 90 days. For this, a sample of the device weighing 0.5 g was dissolved in 20 mL of saline with vigorous stirring. A series of dilutions were made from the resulting suspension, after which it was inoculated onto the Lactobacagar medium. Then the colonies were counted.

The survival rate of bacteria during the production process was determined as the ratio of the number of viable bacteria and the introduced bacteria during the production process. For examples 1-8 and 16-18, the survival rate was 30-70%.

The survival rate of lactobacilli during storage was determined as the ratio of the number of viable microorganisms in a certain period to the number of viable microorganisms immediately after producing the device. The count of viable bacteria for devices from examples 1-8 was 92-105% when stored for 90 days at +2-8 °C, and 84-98% when stored at +25 °C.

### Antimicrobial activity of devices containing lactobacilli

Antimicrobial activity against *C*. *albicans, E. coli, G. vaginalis* was studied after storing samples for 90 days. A sample weighing 0.5 g was dissolved in 10 mL of MRS medium with the addition of 0.05% cysteine. The resulting culture was incubated anaerobically at 37 °C for 24 hours. To obtain a cell-free supernatant (CFS), the cell culture was centrifuged at 5500 rpm for 10 min and the supernatants were collected, after which they were filtered through Millex^{®} GP filters, 0.22 µm Merck Millipore Cork, Ireland.

CFS was added to suspensions of microorganisms *C*. *albicans, E. coli, G. vaginalis* at concentrations of 2.5×10⁵ CFU/mL and incubated for 24 and 48 hours. The microorganism count was determined turbodimetrically at 600 nm. Inhibition % was determined relative to control samples without the addition of CFS. Inhibition % was >90% against *E. coli, G. vaginalis* and >80% against *C*. *albicans.* The resulting suspensions of microorganisms were inoculated onto appropriate media and incubated for 48 hours, after which colonies were counted before and after the addition of CFS. All devices from examples 1-8 showed a decrease in the number of colonies by more than 3 orders of magnitude, which indicates not only the bacteriostatic, but also the bactericidal activity of CFS obtained after culturing samples of the produced devices.

### Antimicrobial activity of devices containing drug substances

The antimicrobial activity of the products was studied against *C*. *albicans, E. coli, G. vaginalis.* For this, a standard test procedure for determining the resistance of microorganisms to antibiotics was adapted. For this, cylindrical samples of devices containing an antimicrobial agent were cut using a scalpel into disks with a diameter of 15 mm and a thickness of about 2 mm. A device according to Example 13, to which no antimicrobial component was added, was also produced, acted as a placebo, and was also cut into disks.

Suspensions of microorganisms *C*. *albicans, E. coli, G. vaginalis* at concentrations of 1×10⁶ CFU/mL were inoculated onto appropriate media in Petri dishes. Cut disks of devices containing an antimicrobial component were placed in the center of each dish. Placebo samples not containing the antimicrobial component were also placed in several dishes. Further, the dishes were incubated for 48 hours, after which the antimicrobial effect of the devices was assessed by the diameter of the growth zone around the disk. The cultures grew evenly over the entire area of the Petri dish; the presence of a placebo disk in the middle of the dish did not lead to any significant change in the growth pattern. In the case of devices with an antimicrobial component, a zone was formed around the disc, in which the growth of microorganisms was suppressed. The diameter of this zone served as a measure of the magnitude of antimicrobial activity. Antimicrobial activity against *G*. *vaginalis* increased in the series of fluconazole< metronidazole< chlorhexidine< miramistin< clindamycin. Antimicrobial activity against *E. coli* increased in the series of fluconazole< clindamycin< chlorhexidine< miramistin< metronidazole. Antimicrobial activity against *C*. *albicans* increased in the series of metronidazole< clindamycin< chlorhexidine< miramistin< fluconazole.

### Determination of device porosity using optical microscopy

To study the internal structure and porosity of the device, dry samples of the device were cut with a razor and examined under an optical microscope. All studied devices of all presented examples had a porous structure, with open, mutually penetrating pores. 90% of the pore sizes calculated from the distance between the nearest structural elements of the device were in the range of 5 to 100 µm and practically did not differ between different devices.

### Modeling the adhesion ability of the material to the epithelial walls

To study the adhesion ability of materials to the walls of the vaginal epithelium, the inclined plate method was used. For this, a thin layer (5 mm) of a solution containing 10% (wt.) acrylamide, 0.1% methylenebisacrylamide and 0.1% potassium peroxodisulfate and tetramethylethylenediamine was poured into a flat mold and polymerized at room temperature. Samples of the device were cut into thin plates about 5 mm thick and placed in a saline solution for hydration. After the formation of acrylamide gel, the gel was wetted with saline and various samples were placed on it and the plate was placed in an incubator at 37 °C. After equilibration for 20 minutes, the plate was tilted at an angle from 30 to 90 degrees and the rate of flowing or rolling of the sample was observed. Commercially available suppositories, 1% starch solution, and samples of the device from Example 9 obtained with and without the addition of hyaluronic acid were used as control samples. As a measure of adhesive ability, the angle at which the sample was observed to slide or flow 10 cm along the surface within 1 minute was determined. The angle was 45 degrees for molten suppositories, 30 degrees for 1% starch solution, 45 degrees for the device from example 9 obtained without the addition of hyaluronic acid, 60 degrees for the device from example 9 obtained with the addition of hyaluronic acid.

Alternatively, a sheet of acrylamide gel was removed from the mold, wetted with saline, samples of the same weight of suppository, 1% starch gel, sample from Example 9 with or without the addition of hyaluronic acid were placed in the middle, the sheet was rolled into a tube and placed in an incubator at 37 °C in vertical position, simulating placement of the sample into the vagina. The time it took for the contents of the tube to leaked of the tube was observed. The 1% starch solution leaked within 30 minutes; the suppository sample leaked within 1 hour. No leakage or falling out of samples from example 9, regardless of the presence of hyaluronic acid, occurred within 4 hours.

### Comparison of lactobacilli release from different dosage forms

To compare the release of lactobacilli from different dosage forms, commercially available vaginal tablets, commercially available vaginal gelatin capsules, and the sample from Example 4 containing the same quantity of lactobacilli were studied. Vaginal tablets, capsules and the sample were placed in a vial and 3 mL of saline was added. The sample from example 4 was pre-hydrated in saline, simulating the method of application, and crushed with gentle pressure. Further, they were placed on a shaker at 20 rpm and 37 °C. After 1 hour, 0.5 mL of the solution was taken and the concentration of released lactobacilli was determined by inoculating onto lactic agar with an accuracy of 1 order of magnitude. In the case of tablets, swelling and partial disintegration of the tablet were observed within 1 hour. The count of detected bacteria was 10⁶ CFU. In the case of capsules, within 1 hour, deformation and sticking of the capsule and its adherence to the wall were observed, practically no disintegration and no visible signs of release of a large volume of contents were observed. The count of detected bacteria was 10⁴ CFU. The sample from example 4 was a gel slurry. The count of detected bacteria was 10⁷ CFU.

### Mechanical strength (breaking stress limit during compression crushing)

To study the limit of breaking stress during compression crushing, a dry cylindrical sample of the device was placed on a horizontal surface. A glass Petri dish was placed on top, on which a weight of 0.1 to 1.0 kg was placed, and the system was kept at room temperature for 1 hour. The limit of the onset of destruction was determined by the appearance of clearly visible cracks.

Thus, in comparison with known from the prior art vaginal gel preparations, vaginal tablets and suppositories based on prebiotics and hard capsules with lyophilized lactobacilli, drugs for the treatment of infectious diseases of the female reproductive system, as well as drugs for restoring microflora as part of two-component therapy for bacterial vaginosis, the claimed invention:
1) Provides the possibility of therapy with vaginal strains of microorganisms most compatible with the administration environment, including personalized strains of microorganisms;
2) Provides high viability of microorganisms during production and storage at room temperature;
3) The biopolymer matrix includes components that are a selective nutrient substrate for lactobacilli of the vaginal flora, but is not suitable for the development of pathogenic flora;
4) Provides rapid hydration of the device upon wetting and effective (quantity over time) release of drug substances and living microorganisms of therapeutic agents after intravaginal administration;
5) Provides rapid achievement of physiological vaginal pH after administration of the device;
6) The cryostructured polysaccharide matrix of the device, upon hydrated, softens and deforms to a soft gel between the vaginal walls, which reduces discomfort after insertion;
7) The device has the shape of a hygienic tampon familiar to patients and is easy to insert. After insertion, the contents do not leak out even if the patient takes a vertical position immediately after insertion, which is confirmed by model experiments.

Compared to the prototype, the claimed device provides a more uniform distribution of the drug throughout the vaginal mucosa, and also contains, in contrast to the neutral filler - mannitol, a selective substrate to create optimal conditions for vaginal lactobacilli and prevent a decrease in their population during therapy.

**Table 3.**

| Example No. | Structural polysaccharide | | Selective substrate | | Mucoadhesive component | | pH modifier (Organic acid) | | pH of the medium | Active ingredient | |
|---|---|---|---|---|---|---|---|---|---|---|---|
| | Name | Concentration, wt% | Name | Concentration, wt% | Name | Concentration, wt% | Name | Concentration, wt% | | Name | Concentration, wt% |
| 1 | Corn starch | 6.0 | Inulin | 3.5 | Hydroxypropyl cellulose | 2.0 | Ascorbic acid | 3.0 | 4.7 | *Lactobacillus crispatus* | 0,1 * |
| 2 | Potato starch | 10.0 | Maltodextrin | 4.0 | Xanthan gum | 1.5 | Succinic acid | 2.5 | 4.5 | *Lactobacillus crispatus* | 1,0* |
| 3 | Potato starch / Corn starch (50/50 wt%) | 10.0 | Maltodextrin | 4.0 | Hyaluronic acid | 1.5 | Succinic acid | 2.5 | 4.5 | *Lactobacillus crispatus* | 1.0* |
| 4 | Tapioca starch | 8.5 | Oligo-fructose | 2.0 | Carboxymethyl cellulose | 3.0 | Pyruvic acid | 1.0 | 5.1 | *Lactobacillus gasseri* | 0.4* |
| 5 | Wheat starch | 7.0 | Isomaltooligosaccharide | 3.0 | Hyaluronic acid | 1.0 | Malic acid | 2.0 | 4.6 | *Lactobacillus gasseri* | 0.15* |
| 6 | Sago starch | 9.0 | Maltodextrin | 2.5 | Hydroxypropyl cellulose | 2.5 | Lactic acid | 1.5 | 4.9 | *Lactobacillus plantarum* | 0.3* |
| 7 | Tapioca starch | 8.0 | Inulin | 3.5 | Carboxymethyl cellulose | 2.5 | Ascorbic acid | 2.5 | 3.8 | *Lactobacillus plantarum* | 0.8* |
| 8 | Waxy corn starch | 6.5 | Oligo-fructose | 3.7 | Hydroxypropyl cellulose | 2.3 | Succinic acid | 2.2 | 4.2 | *Lactobacillus crispatus*/ *Lactobacillus gasseri*/ *Lactobacillus plantarum* | 0.9* |
| 9 | Potato starch | 9.5 | Maltodextrin | 3.2 | Hyaluronic acid | 1.3 | Pyruvic acid | 1.2 | 4.0 | *Lactobacillus Lactobacillus gasseri*/ *Lactobacillus plantarum* | 1.0* |
| 10 | Potato starch | 10.5 | Maltodextrin | 4.2 | Hyaluronic acid | 1.5 | Ascorbic acid | 1.5 | 4.1 | Chlorhexidine digluconate | 0.15 |

**Table 3 (continued)**

| Example No. | Structural polysaccharide | | Selective substrate | | Mucoadhesive component | | pH modifier (Organic acid) | | pH of the medium | Active ingredient | |
|---|---|---|---|---|---|---|---|---|---|---|---|
| | Name | Concentration, wt% | Name | Concentration, wt% | Name | Concentration, wt% | Name | Concentration, wt% | | Name | Concentration, wt% |
| 11 | Potato starch | 9.6 | Maltodextrin | 3.7 | Hyaluronic acid | 1.5 | Ascorbic acid | 1.5 | 4.0 | Miramistin hydrochloride | 0.01 |
| 12 | Potato starch | 9.5 | Maltodextrin | 3.2 | Hyaluronic acid | 1.3 | Ascorbic acid | 1.5 | 4.2 | Metronidazole | 6.0 |
| 13 | Potato starch | 9.5 | Maltodextrin | 3.2 | Guar gum | 1.3 | Ascorbic acid | 1.5 | 3.8 | Clindamycin, hydrochloride | 1.2 |
| 14 | Dextrin | 9.5 | Maltodextrin | 3.2 | Hyaluronic acid | 1.3 | Ascorbic acid | 1.5 | 3.9 | Fluconazole | 1.9 |
| 15 | Potato starch | 9.5 | Maltodextrin | 3.2 | Hyaluronic acid | 1.3 | | 1.5 | 4.1 | Tenofovir | 1.0 |
| 16 | Amylopectin | 6.5 | Lactulose | 3.7 | Hyaluronic acid | 2.3 | Succinic acid | 2.2 | 4.2 | *Lactobacillus crispatus* | 0.9* |
| 17 | Amylopectin | 6.5 | Raffinose | 3.7 | Hyaluronic acid | 2.3 | Ascorbic acid | 2.2 | 4.2 | *Lactobacillus crispatus* | 0.9* |
| 18 | Polydextrose | 6.5 | Glycogen | 3.7 | Hyaluronic acid | 2.3 | Pyruvic acid | 2.2 | 4.2 | *Lactobacillus crispatus* | 0.9* |
| 19 | Potato starch | 10.0 | Maltodextrin | 4.0 | Hyaluronic acid | 1.5 | Ascorbic acid | 2.5 | 4.3 | *Escherichia coli* lysate | 0.08 |
| 20 | Corn starch | 10.0 | Maltodextrin | 4.0 | Hyaluronic acid | 1.5 | Ascorbic acid | 2.5 | 4.5 | Estriol (micronized) | 0.0025 |
| 21 | Potato starch / Corn starch (50/50 wt%) | 10.0 | Maltodextrin | 4.0 | Hyaluronic acid | 1.5 | Ascorbic acid | 2.5 | 4.1 | Fluorouracil | 0.5 |
| 22 | Potato starch / Corn starch (50/50 wt%) | 10.0 | Maltodextrin | 4.0 | Hyaluronic acid | 1.5 | Ascorbic acid | 2.5 | 4.5 | | - |

**Table 3 (continued)**

| Exampl e No. | Structural polysaccharide | | Selective substrate | | Mucoadhesive component | | pH modifier (Organic acid) | | pH of the mediu m | Active ingredient | |
|---|---|---|---|---|---|---|---|---|---|---|---|
| | Name | Concentration, wt% | Name | Concentration, wt% | Name | Concentratio n, wt% | Name | Concentration, wt% | | Name | Concentration, wt% |
| 23 | Potato starch | 10.0 | - | - | Hyaluronic acid | 1.5 | Ascorbic acid | 2.5 | 4.5 | - | - |
| 24 | Corn starch | 10.0 | - | - | Hyaluronic acid | 1.5 | - | - | - | - | - |
| 25 | Potato starch / Corn starch (50/50 wt%) | 10.0 | Maltodextrin | 4.0 | Hyaluronic acid | 1.5 | Ascorbic acid | 2.5 | 4.5 | Miramistin | Swelling in 0.01% miramistin solution |
| 26 | Potato starch | 9.5 | Maltodextrin | 3.2 | Hyaluronic acid | 1.3 | Ascorbic acid | 1.5 | 3.8 | Clindamycin, hydrochloride | 1.2 |
| | | | | | | | | | | Miramistin | Swelling in 0.01% miramistin solution |

| | | | | | | | | | | | |
|---|---|---|---|---|---|---|---|---|---|---|---|
| * When the content of living microorganisms in the dried powder is 1×10¹⁰ CFU/g | | | | | | | | | | | |

**Table 4.**

| Example No. from Table 3 | Initial composition freezing regimen | | Characteristics of the resulting medical device | | | | Cell survival | Swelling capacity | Hydration time | Release rate | Porosity | Moisture content, % | Mechanical strength |
|---|---|---|---|---|---|---|---|---|---|---|---|---|---|
| | Temperature, °C | Time, h | Form | Length, mm | Diameter, mm | Weight, g | lactobacilli, % | Swollen weight/dry weight | Time to maximum weight, min | Time to release >80%, min | Specific pore density, g/cm³ | | Breaking stress limit during compressive crushing, N |
| 1 | -15 | 18 | Cylindrical rod with rounded end | 40 | 12 | 0.69 | 33 | 6.2 | 2.3 | 50 | 0.10 | 4 | 2 |
| 2 | -30 | 3 | | 50 | 15 | 1.7 | 45 | 5.0 | 2.7 | 60 | 0.19 | na | na |
| 3 | -20 | 10 | | 50 | 15 | 1.65 | 52 | 5.4 | 2.8 | 60 | 0.15 | na | 10 |
| 4 | -20 | 10 | | 45 | 10 | 0.54 | 37 | 6.4 | 3.8 | 30 | 0.13 | na | na |
| 5 | -18 | 12 | | 55 | 8 | 0.35 | 28 | 7.0 | 1.5 | 25 | 0.16 | na | na |
| 6 | -24 | 8 | | 20 | 5 | 0.12 | 44 | 4.0 | 2.4 | 40 | 0.17 | na | na |
| 7 | -24 | 8 | | 50 | 9 | 0.57 | 53 | 5.4 | 3.6 | 50 | 0.16 | na | na |
| 8 | -22 | 9 | | 45 | 15 | 1.2 | 63 | 6.1 | 1.7 | 47 | 0.16 | 7 | 5 |
| 9 | -19 | 18 | | 55 | 14 | 1.3 | 32 | 6.0 | 1.9 | 38 | 0.19 | na | na |
| 10 | -19 | 18 | | 55 | 14 | 1.5 | - | 5.0 | 3.6 | 5 | 0.16 | na | na |
| 11 | -19 | 18 | | 54 | 14 | 1.3 | - | 6.1 | 2.1 | 5 | 0.3 | na | na |
| 12 | -30 | 18 | | 100 | 15 | 2.5 | - | 6.0 | 4.7 | 10 | 0.17 | 5 | 10 |
| 13 | -19 | 18 | | 54 | 14 | 1.4 | - | 5.5 | 4.0 | 5 | 0.22 | na | na |
| 14 | -19 | 18 | | 54 | 14 | 1.4 | - | 5.2 | 4.3 | 10 | 0.17 | na | na |
| 15 | -19 | 18 | | 54 | 14 | 1.3 | - | 5.4 | 5.0 | 5 | na | 3 | na |
| 16 | -22 | 9 | | 45 | 15 | 1.2 | 46 | 6.3 | 2.4 | 55 | na | na | na |
| 17 | -22 | 9 | | 45 | 15 | 1.2 | 39 | 5.9 | 3.0 | 60 | na | 9 | 6 |
| 18 | -22 | 9 | | 45 | 15 | 1.2 | 29 | 6.0 | 0.5 | 60 | na | na | na |
| 19 | -20 | 10 | | 50 | 15 | 1.5 | - | 5.3 | na | na | na | na | na |
| 20 | -20 | 10 | | 50 | 15 | 1.5 | - | 5.7 | na | na | na | na | na |
| 21 | -20 | 10 | | 50 | 15 | 1.5 | - | 5.5 | na | na | na | na | na |

**Table 4(continued)**

| Example No. from Table 3 | Initial composition freezing regimen | | Characteristics of the resulting medical device | | | | Cell survival | Swelling capacity | Hydration time | Release rate | Porosity | Moisture content, % | Mechanical strength |
|---|---|---|---|---|---|---|---|---|---|---|---|---|---|
| | Temperature, °C | Time, h | Form | Length, mm | Diameter, mm | Weight, g | lactobacilli, % | Swollen weight/dry weight | Time to maximum weight, min | Time to release >80%, min | Specific pore density, g/cm³ | | Breaking stress limit during compressive crushing, N |
| 22 | -20 | 10 | | 50 | 15 | 1.5 | | 5.0 | 2.1 | na | na | na | na |
| 23 | -20 | 10 | | 50 | 15 | 1.2 | | 7.1 | 1.8 | na | na | na | na |
| 24 | -20 | 10 | | 50 | 15 | 1.0 | | 8.0 | 2.5 | na | na | na | na |
| 25 | -20 | 10 | | 50 | 15 | 1.5 | na | na | na | na | na | na | na |
| 26 | -19 | 18 | | 54 | 14 | 1.4 | na | na | na | na | na | na | na |

## Claims

1. A composition for producing an intravaginal delivery device for an active ingredient, comprising at least one structural polysaccharide selected from the group consisting of polysaccharides biodegradable in a vaginal cavity, at least one mucoadhesive component, and at least one pH modifier, which is an organic acid, taken in the following quantities per 1 mL of water or aqueous solution:
structural polysaccharide - 40-100 mg,
mucoadhesive component - 10-40 mg,
organic acid - 10-30 mg.

2. The composition according to claim 1, **characterized in that** the structural polysaccharide is selected from the group of polysaccharides, glucose molecules of which are linked together by glycosidic bonds α-(1→4) and α-(1→6).

3. The composition according to claim 1, **characterized in that** the structural polysaccharide is selected from the group consisting of starch, amylopectin, dextrins, polydextrose.

4. The composition according to claim 1, **characterized in that** the mucoadhesive component is selected from the group consisting of hydroxypropylcellulose, carboxymethylcellulose, hyaluronic acid, guar gum, xanthan gum.

5. The composition according to claim 1, **characterized in that** the organic acid is an acid suitable for intravaginal administration, including ascorbic, pyruvic, malic, lactic or succinic acid.

6. The composition **characterized in that** it further comprises a selective microbial substrate taken in an amount of 10-40 mg per 1 mL of water or aqueous solution selected from the group consisting of oligosaccharides biodegradable in the vaginal cavity.

7. The composition according to claim 6, **characterized in that** the selective substrate is selected from the group of substrates intended for intravaginal lactobacilli.

8. The composition according to claim 6, **characterized in that** the selective substrate is selected from the group of oligosaccharides, the chain of which contains 2-20 monosaccharide units.

9. The composition according to claim 6, **characterized in that** the selective substrate is selected from the group consisting of lactulose, raffinose, maltodextrin, oligofructose, isomaltooligosaccharide, inulin, glycogen.

10. The composition according to claim 1, **characterized in that** it further comprises at least one active ingredient in a therapeutically effective amount.

11. The composition according to claim 10, **characterized in that** the active ingredient is selected from the group consisting of antibacterial agents, including macrophages, and/or antiseptic agents, and/or antifungal agents, and/or antiretroviral agents, as well as hormones, cytostatic agents, vaccines intended for intravaginal administration.

12. The composition according to claim 11, **characterized in that** antibacterial agents, and/or antiseptic agents, and/or antifungal agents, and/or antiretroviral agents, or hormones or cytostatic agents or vaccines are taken in the following amount per 1 mL of water or an aqueous solution of the composition:
antibacterial agents - 5-50 mg,
antiseptic agents - 0.1-10 mg,
antifungal agents - 5-50 mg,
antiretroviral agents - 10-50 mg,
hormones - 0.02-2 mg,
cytostatic agents - 5-50 mg, vaccines - 2-1000 µg.

13. The composition according to claim 6, **characterized in that** it further comprises at least one active ingredient in a therapeutically effective amount selected from the group consisting of probiotic microorganisms, including lactobacilli, consortia of probiotic microorganisms for intravaginal administration.

14. The composition according to claim 13, **characterized in that** probiotic microorganisms are taken in an amount of 1×10⁶ - 1×10⁹ CFU per 1 mL of water or aqueous solution.

15. An intravaginal delivery device for an active ingredient produced from the composition according to claim 1, which is a solid volumetric body with a macroporous structure, comprising after freezing and freeze-drying at least one structural polysaccharide selected from the group consisting of polysaccharides biodegradable in the vaginal cavity at least one mucoadhesive component, at least one pH modifier, which is an organic acid, in the following amount per 1 g of the device: structural polysaccharide - 400-700 mg, mucoadhesive component - 150-300 mg, organic acid - 50-100 mg,
with the total content of all ingredients not exceeding 1 gram.

16. The device according to claim 15, **characterized in that** the moisture content is not more than 9%.

17. The device according to claim 15, **characterized in that** the macroporous structure is **characterized by** an average pore size of 5-100 µm and a specific density of 0.1-0.3 g/cm³.

18. The device according to claim 15, **characterized in that** it is designed to absorb water or aqueous solutions for not more than 5 minutes in the amount required to fill the internal volume of macropores with the formation of a hydrogel that retains its original shape, **characterized by** an ultimate stress limit during compression crushing sufficient for non-destructive introduction of hydrogel into the vaginal cavity.

19. The device according to claim 18, **characterized in that** the hydrogel has an ultimate stress limit during compression crushing of 0.2-1.0 N.

20. The device according to claim 15, **characterized in that** it is designed to absorb water or aqueous solutions of at least 3 mL per gram of the device or at not less than 70% of the weight of the device saturated with water.

21. The device according to claim 15, **characterized in that** the structural polysaccharide is selected from the group of polysaccharides, glucose molecules of which are linked together by glycosidic bonds α-(1→4) and α-(1→6).

22. The device according to claim 15, **characterized in that** the structural polysaccharide is selected from the group consisting of starch, amylopectin, dextrins, polydextrose.

23. The device according to claim 15, **characterized in that** the mucoadhesive component is selected from the group consisting of hydroxypropylcellulose, carboxymethylcellulose, hyaluronic acid, guar gum, xanthan gum.

24. The device according to claim 15, **characterized in that** the organic acid is an acid suitable for intravaginal administration, including ascorbic, pyruvic, malic, lactic or succinic acid.

25. The device according to claim 15, **characterized in that** it further comprises a selective microbial substrate taken in an amount of 100-250 mg per 1 g of the device.

26. The device according to claim 25, **characterized in that** the selective substrate is selected from the group of substrates intended for intravaginal lactobacilli.

27. The device according to claim 25, **characterized in that** the selective substrate is selected from the group of oligosaccharides, the chain of which contains 2-20 monosaccharide units.

28. The device according to claim 25, **characterized in that** the selective substrate is selected from the group consisting of lactulose, raffinose, maltodextrin, oligofructose, isomaltooligosaccharide, inulin, glycogen.

29. The device according to claim 25, **characterized in that** it further comprises at least one active ingredient in a therapeutically effective amount selected from the group consisting of probiotic microorganisms, including lactobacilli, consortia of probiotic microorganisms for intravaginal administration.

30. The device according to claim 29, **characterized in that** probiotic microorganisms are taken in an amount of 1×10⁶ - 1×10⁹ CFU per 1 mL of water or aqueous solution.

31. The device according to claim 15, **characterized in that** it further comprises the active ingredient in a therapeutically effective amount selected from the group consisting of antibacterial agents, including macrophages, and/or antiseptic agents, and/or antifungal agents, and/or antiretroviral agents, as well as hormones, cytostatic agents, vaccines intended for intravaginal administration.

32. The device according to claim 31, **characterized in that** antibacterial agents, and/or antiseptic agents, and/or antifungal agents, and/or antiretroviral agents, or hormones or cytostatic agents or vaccines are taken in the following amount per 1 g of the device:
antibacterial agents - 30-250 mg,
antiseptic agents - 0.5-70 mg,
antifungal agents - 30-250 mg,
antiretroviral agents - 50-250 mg,
hormones - 0.1-15 mg,
cytostatic agents - 3-250 mg,
vaccines - 0.01-10 mg
with the total content of all ingredients not exceeding 1 gram.

33. The device according to claim 15, **characterized in that** it is made in the form of an oblong volumetric body shaped close to a 20 mm to 100 mm long hygienic tampon with a diameter of 5 to 20 mm.

34. A method of producing of the device according to claim 15, comprising the preparation of aqueous solutions of the composition ingredients according to claim 1, mixing thereof, freezing the resulting mixture at a temperature selected from the range from minus 15°C to minus 30°C for 3-18 hours with subsequent freeze-drying.

35. A method for intravaginal delivery of an active ingredient for treating and/or preventing diseases of female reproductive system organs, comprising intravaginal administration according to claim 15.

36. The method according to claim 35, **characterized in that** the device is pre-wetted in water or an aqueous solution before intravaginal administration.

37. The method according to claim 35, **characterized in that** the device is allowed to stand in water or an aqueous solution for up to 5 minutes.

38. The method according to claim 35, **characterized in that** the device is administered with an applicator.

39. The method according to claim 35, **characterized in that** the device is used for treating and/or preventing bacterial vaginosis; purulent inflammatory diseases, sexually transmitted infections, benign and malignant neoplasms in the genital organs, fibroids, hyperplastic and dystrophic changes in the cervix, sexual dysfunction, reproductive dysfunction, menstrual dysfunction.
